# EUROPEAN PATENT APPLICATION

(11) **EP 4 458 388 A1**
(43) Date of publication of application: **06.11.2024**
(21) Application number: 22914822.6
(22) Date of filing: 27.12.2022
(51) Int. Cl.: A61M 5/315

(54) **INTERVENTIONAL INJECTION DEVICE AND INTERVENTIONAL INJECTION SYSTEM**

(30) Priority: 30.12.2021 CN 202111660519
(71) Applicant: Hangzhou Deke Medtech Co., Ltd., Hangzhou, Zhejiang 310052 (CN)
(72) Inventor: SHAO, Nan, Hangzhou, Zhejiang 310052 (CN); LI, Yang, Hangzhou, Zhejiang 310052 (CN); ZHUANG, Zhenping, Hangzhou, Zhejiang 310052 (CN); ZHU, Peng, Hangzhou, Zhejiang 310052 (CN)
(74) Representative: Zaboliene, Reda
(86) International application number: PCT/CN2022/142504
(87) International publication number: WO 2023/125570

(57) **Abstract**

The present disclosure discloses an interventional injection device and an interventional injection system. The interventional injection device includes an operating handle, a sheath and an injection member. The operating handle includes a housing and a driving assembly. The driving assembly includes a driving knob that is rotatably connected to the housing and a driving rod that is movably inserted into the housing and threaded to the driving knob. A proximal end of the sheath is fixedly connected to the housing. The injection member is movably inserted in the sheath. The injection member includes a first channel and an injection needle connected to a distal end of the first channel in a tight seal, and a proximal end of the injection member is fixedly connected to the driving rod. The driving rod is configured to move axially by rotation of the driving knob relative to the housing so as to drive the injection member to move and thus drive the injection needle to penetrate into a target tissue region. Since the driving rod and the driving knob are threaded to each other, the rotational motion is converted into a linear motion for advancing the injection needle. The driving knob is rotated so that the advance speed of the needle can be controlled more accurately and stably.

## Description

### TECHNICAL FIELD

The present disclosure relates to the technical field of medical devices, and in particular to an interventional injection device and an interventional injection system.

### DESCRIPTION OF THE PRIOR ART

Heart failure (HF) refers to heart circulatory disturbances caused by congestion in the venous system and insufficient perfusion in the arterial system because venous returning blood cannot be fully discharged from the heart due to cardiac systolic dysfunction and diastolic dysfunction. A main manifestation of heart failure worsening and pathological remodeling is the changes in left ventricular morphology and structure, such as ventricular enlargement, hypertrophy, and spherical dilation, which further aggravate the patient's clinical symptoms.

With the development of medical technology, heart failure treatment methods such as injecting therapeutic agents for myocardial repair, such as alginate hydrogel, into the ventricular wall have emerged. Extensive clinical investigations have shown that the alginate hydrogel injected into the ventricular wall can change the geometric structure of the myocardium and the myocardial tension, improving cardiac function, and delay or reverse the progression of heart failure in patients with left ventricular enlargement, significantly improving cardiac function.

The myocardial repair injection device is a medical device used to inject alginate hydrogel for the treatment of heart failure into the ventricular wall. The myocardial repair injection device advances the injection needle through its operating handle. The operating handles of existing myocardial repair injection devices mostly control the injection needle to penetrate the ventricular wall based on ejection or push-pull techniques. However, the ejection and push-pull techniques have poor controllability, and it is difficult to advance the needle accurately and stably.

### SUMMARY OF THE DISCLOSURE

The technical object of the present disclosure is to provide an interventional injection device and an interventional injection system, to solve the problems of poor controllability and being difficult to advance the needle accurately and stably, caused by the existing ejection or push-pull techniques of driving the injection needle.

In order to achieve the above objective, the present disclosure adopts the following technical solutions.

In a first aspect, the present disclosure provides an interventional injection device, including an operating handle, a sheath and an injection member; the operating handle includes a housing and a driving assembly; wherein the driving assembly includes a driving knob that is rotatably connected to the housing and a driving rod that is movably inserted into the housing and threaded to the driving knob; a proximal end of the sheath is fixedly connected to the housing; the injection member is movably inserted in the sheath, the injection member includes a first channel and an injection needle connected to a distal end of the first channel in a tight seal, and a proximal end of the injection member is fixedly connected to the driving rod; and the driving rod is configured to move axially by rotation of the driving knob relative to the housing so as to drive the injection member to move and thus drive the injection needle to penetrate into a target tissue region.

In the following, several alternatives are provided, but merely as further additions or preferences, instead of as additional limitations to the above-mentioned technical solution. Without technical or logical contradiction, the alternatives can be combined with the above-mentioned technical solution, individually or in combination.

Optionally, the driving knob defines a threaded hole extending axially, an inner wall of the threaded hole is provided with a female thread, an outer wall of the driving rod is provided with a male thread, and the driving rod passes through the threaded hole so that the female thread is threaded with the male thread.

Optionally, the driving rod includes:
a rod body, the male thread is provided on the rod body; and
a limiting portion, which radially protrudes from an outer wall of the rod body;
wherein an inner wall of the housing is provided with a blocking part which is configured to block the limiting portion so as to limit axial movement of the driving rod.

Optionally, the driving rod further includes an indicator portion which radially protrudes from the outer wall of the rod body.

The housing is provided with a viewing window to show the indicator portion, and the housing is provided with a scale for indicating position of the indicator portion.

Optionally, a damping structure is provided between the driving knob and the housing.

Optionally, the damping structure includes a damping ring and a rib in an interference fit with the damping ring, and one of the damping ring and the rib is provided on the housing, and the other is provided on the driving knob.

Optionally, a part of the driving knob extends into an interior of the housing and is provided with a circumferential groove, and the damping ring is at least partially received in the circumferential groove; and the rib protrudes inward on an inner wall of the housing, and the rib press the damping ring.

Optionally, the interventional injection device further includes an injection interface seat located outside the housing, a proximal end of the driving rod is fixedly connected to the injection interface seat, and a proximal end of the injection member is fixedly connected to the injection interface seat.

Optionally, the driving rod defines a connection through-hole extending in an axial direction, a first protection tube is arranged in the connection through-hole and extends out of the connection through-hole, and the injection member is inserted into the first protection tube and the connection through-hole.

Optionally, the operating handle further includes a hemostatic valve assembly fixedly installed in the housing, and a distal end of the hemostatic valve assembly is fixedly connected to the proximal end of the sheath, wherein the hemostatic valve assembly includes a sealing gasket, a second protection tube passes through the sealing gasket, the second protection tube is connected to the sealing gasket in a tight seal, and the injection member passes through the second protection tube.

Optionally, the injection member further includes a second channel that is isolated from the first channel.

Optionally, the injection interface seat includes:
a first branch interface selectively connected to a proximal end of the first channel; and
a second branch interface selectively connected to a proximal end of the second channel;
wherein the second branch interface and the first branch interface are isolated from each other.

Optionally, the injection member further includes an inner tube and an outer tube surrounding the inner tube; a lumen of the inner tube forms the first channel, and the second channel is defined between an inner wall of the outer tube and an outer wall of the inner tube; and at least the injection needle is exposed outside a distal end of the outer tube.

Optionally, a distal end of the sheath is provided with a head for abutting the target tissue region, the head defines an injection orifice, and a distal end of the second channel is communicated with the injection orifice.

Optionally, the injection orifice has an axial direction perpendicular to an axial direction of the sheath, and a plurality of injection orifices are distributed on the head at intervals in a circumferential direction.

Optionally, a third channel is defined between an inner wall of the sheath and an outer wall of the outer tube, a distal end surface of the head has a needle outlet, and a distal end of the third channel is communicated with the needle outlet and the injection orifice.

Optionally, the operating handle further includes a hemostatic valve assembly fixedly provided in the housing, and a proximal end of the third channel is communicated with the hemostatic valve assembly.

Optionally, the interventional injection device further includes a feedback mechanism, the feedback mechanism including:
a middle piece fixedly connected to the housing of the operating handle, an outer wall of the middle piece being provided with a first abutment portion;
a sleeving tube movably arranged around the middle piece, an inner wall of the sleeving tube is provided with a second abutment portion, wherein in an initial state, a proximal end of the sleeving tube contacts a distal end of the housing, and the second abutment portion is located on a proximal side of the first abutment portion; and
a spring element, one end of the spring element abuts the first abutment portion, and the other end of the spring element abuts the second abutment portion;
wherein when the distal end of the sheath abuts the target tissue region, the middle piece is forced to press the spring element and the middle piece moves proximally relative to the sleeving tube, generating a gap between the proximal end of the sleeving tube and the distal end of the housing.

Optionally, the spring element is arranged around the middle piece and received in the sleeving tube.

Optionally, the first abutment portion is a radial step radially provided on the middle piece.

Optionally, the second abutment portion is a radial step radially provided on the inner wall of the sleeving tube.

In a second aspect, the present disclosure provides an interventional injection system, including a bending device and the interventional injection device as mentioned above. The bending device includes a bendable sheath and a bending handle for controlling the bendable sheath. The sheath is movably inserted in the bending device.

Optionally, the interventional injection system further includes an adjusting device, and the adjusting device includes:
a connecting piece, fixedly connected to a proximal end of the bending handle;
a guide tube, inserted into the connecting piece and rotatably connected to the connecting piece, the sheath being movably inserted into the guide tube;
a middle piece, movably arranged around the guide tube and fixedly connected to the housing; and
an adjusting piece, movably provided on the middle piece and configured to directly or indirectly lock or unlock the guide tube in a radial direction of the middle piece to prevent or allow axial movement of the middle piece relative to the guide tube, thereby preventing or allowing axial movement of the operating handle relative to the bending handle.

Optionally, the interventional injection system further includes an auxiliary support.

The outer wall of the middle piece is provided with a first abutment portion.

The adjusting device further includes:
a sleeving tube fixedly connected to the auxiliary support and movably arranged around the middle piece, an inner wall of the sleeving tube being provided with a second abutment portion, wherein in an initial state, a proximal end of the sleeving tube contacts a distal end of the housing; and
a spring element provided between the middle piece and the sleeving tube, wherein one end of the spring element abuts the first abutment portion, and the other end of the spring element abuts the second abutment portion;

When a distal end of the sheath abuts the target tissue region, the middle piece is forced to press the spring element and the middle piece moves proximally relative to the sleeving tube, generating a gap between the proximal end of the sleeving tube and the distal end of the housing.

In the interventional injection device and interventional injection system provided by the present disclosure, the driving assembly for driving the injection member includes a driving knob that is rotationally connected to the housing of the handle and a driving rod that is screwed to the driving knob. The driving knob rotates relative to the housing to drive the driving rod to move axially so as to drive the injection member to move, so that the injection needle can be advanced to penetrate into the target tissue region. Since the driving rod and the driving knob are threaded to each other, the rotational motion is converted into a linear motion for advancing the injection needle. The driving knob is rotated so that the advance speed of the needle can be controlled more accurately and stably. Further, the threaded connection has a certain self-locking property, so that backout of the injection needle can be effectively prevented when the injection needle penetrates into the target tissue region, and the stability of the needle advancing process is further improved, improving the accuracy and efficiency of surgery.

### BRIEF DESCRIPTION OF DRAWINGS

FIG. 1 is a schematic perspective view of an assembled interventional injection system according to an embodiment of the present disclosure;
FIG. 2 is a perspective view of an assembled interventional injection device of the interventional injection system shown in FIG. 1;
FIG. 3 is a schematic view of the interventional injection device shown in FIG. 2 from another perspective;
FIG. 4 is a schematic exploded view of the interventional injection device shown in FIG. 2;
FIG. 5 is a partial axial cross-sectional view of the interventional injection device shown in FIG. 2;
FIG. 6 is a side view of a driving rod, a first protection tube, and an injection interface seat of the interventional injection device which are assembled together;
FIG. 7a is a side view of the driving rod and the first protection tube assembled together;
FIG. 7b is an axial cross-sectional view of the driving rod and the first protection tube assembled together;
FIG. 8 is a partially enlarged view of the proximal section of an operating handle of the interventional injection device shown in FIG. 5;
FIG. 9a is a schematic view of a damping ring in the operating handle of the interventional injection device;
FIG. 9b is a cross-sectional view of the damping ring shown in FIG. 9a;
FIG. 10a is a side view of an injection member and the injection interface seat of the interventional injection device which are assembled together;
FIG. 10b is an axial cross-sectional view of the injection member and the injection interface seat of the interventional injection device which are assembled together;
FIG. 11 is an axial cross-sectional view of a hemostatic valve assembly in the operating handle of the interventional injection device;
FIG. 12a is a top view of a sealing gasket in the hemostatic valve assembly;
FIG. 12b is a cross-sectional view of the sealing gasket;
FIG. 13 is a schematic structural view of the distal end of a sheath of the interventional injection device;
FIG. 14a is a schematic view of an adjusting device of the interventional injection system;
FIG. 14b is an axial cross-sectional view of the adjusting device shown in FIG. 14a;
FIG. 15a is a perspective exploded view of an adjustment knob and an adjusting piece of the adjusting device;
FIG. 15b is an axial cross-sectional view of the adjustment knob and the adjusting piece of the adjusting device which are assembled together;
FIGS. 16 to 18 are schematic views showing the process of injecting a myocardial repair substance into the myocardial tissue using the interventional injection system;
FIG. 19 is an axial cross-sectional view of an interventional injection system according to another embodiment of the present disclosure;
FIG. 20 is a cross-sectional view of an adjusting device and an operating handle when a head at the distal end of a sheath in the interventional injection system shown in FIG. 19 does not abut against the target tissue region; and
FIG. 21 is a cross-sectional view of the adjusting device and the operating handle when the head at the distal end of the sheath in the interventional injection system shown in FIG. 19 abuts against the target tissue region.

### DESCRIPTION OF EMBODIMENTS

In order to make the objects, technical solutions and advantages of the embodiments of the disclosure more apparent, the technical solutions according to the embodiments of the disclosure will be described below in detail with reference to the drawings in the embodiments of the disclosure.

It should be understood that terms such as "comprises" and "can comprise" used in this specification specify the presence of disclosed features, operations, or elements, and do not preclude one or more additional features, operations, or elements. In the present disclosure, terms such as "includes" and/or "has" specify the presence of specific characteristics, numbers, operations, elements, components, or combinations thereof, but do not preclude the presence or addition of one or more other characteristics, numbers, operations, elements, components, or combinations thereof.

Furthermore, in the present disclosure, the phrase "and/or" includes any one or more of the listed elements. For example, the phrase "A and/or B" includes "A", "B", or "A and B".

In the present disclosure, expressions including ordinal numbers such as "first" and "second" can be used to modify various elements. However, these elements are not limited by such expressions. For example, such expressions do not limit the order and/or importance of the elements. Such expressions are only intended to distinguish one element from other elements. Similarly, a first element could be termed a second element, and a second element could be termed a first element, without departing from the scope of the present disclosure.

When a component "connects" or is "connected" to another component, it will be understood that the component can directly connect or be directly connected to another component, or connect or be connected to another component through an intermediate component. However, when a component "directly connects" or is "directly connected" to another component, it should be understood that no intermediate component is present between them.

In the field of interventional medical device technology, the proximal end refers to the end close to the operator, and the distal end refers to the end far away from the operator. The axial direction refers to the direction along the central axis of rotation of objects such as cylinders or tubes. The radial direction refers to the direction perpendicular to the axial direction and along the diameter or radius. The circumferential direction refers to the direction around the axis of objects such as cylinders or tubes (perpendicular to the axis and perpendicular to the section radius).

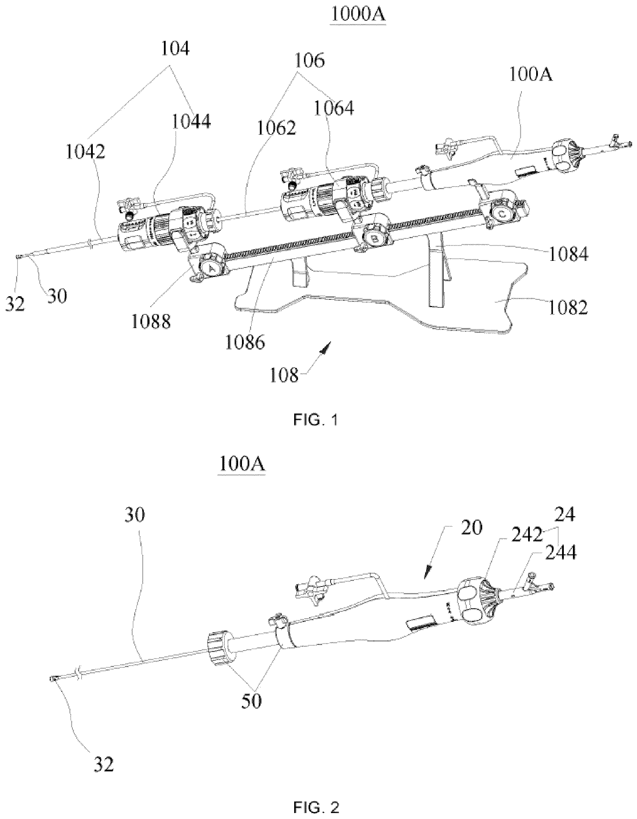

Referring to FIG. 1, an embodiment of the present disclosure provides an interventional injection system 1000A, which can inject a first injectable substance into a target tissue region through different interventional paths to repair or treat the target tissue region.

In this embodiment, the target tissue region is myocardial tissue. The interventional injection system 1000A can be introduced into the heart through a catheter and inject the first injectable substance into the myocardial tissue of a heart failure patient, to repair or treat the heart failure. Preferably, the first injectable substance is, but not limited to, an alginate hydrogel composed of sodium alginate system, calcium alginate system, and sodium chloride solution. The myocardial tissue can be but is not limited to atrial wall, ventricular wall, or interventricular septum, or the like.

It can be understood that the interventional injection system 1000A is also suitable for injection treatment for other target tissue regions.

The interventional injection system 1000A includes an interventional injection device 100A, a guiding device 104, a bending device 106 and an auxiliary support 108. The guiding device 104, the bending device 106, and the interventional injection device 100A are all supported by the auxiliary support 108. The guiding device 104 includes a bendable guiding sheath 1042 and a guide handle 1044 for controlling the guiding sheath 1042. The bending device 106 includes a bendable sheath 1062 and a bending handle 1064 for controlling the bendable sheath 1062. The bendable sheath 1062 is movably inserted into the guide handle 1044 and the guiding sheath 1042. The interventional injection device 100A is movably inserted into the bending device 106 and is configured to penetrate into the target tissue region for injection.

During surgery, the guiding sheath 1042 first enters the patient's body to establish a path for the bendable sheath 1062 and the interventional injection device 100A. The bendable sheath 1062 has good bending and steering performance. By adjusting the bendable sheath 1062, the interventional injection device 100A passing through the bendable sheath 1062 can be adjusted to approach the target tissue region and to a suitable injection site.

The auxiliary support 108 includes a bottom plate 1082, a support post 1084, a guide rod 1086 and three clamping members 1088. The support post 1084 is vertically fixed on the bottom plate 1082. The guide rod 1086 is fixed to an end of the support post 1084 away from the bottom plate 1082. The guide rod 1086 is inclined relative to the bottom plate 1082. The three clamping members 1088 are respectively movable along the guide rod 1086 and positionable on the guide rod 1086. The guide handle 1044, the bending handle 1064 and the interventional injection device 100A each have a clamping position where the corresponding clamping member 1088 can clamp and fix the corresponding component, thereby ensuring the positional stability of the corresponding component on the guide rod 1086. For example, after the guide handle 1044 and the bending handle 1064 are adjusted and fixed on the guide rod 1086 through the corresponding clamping members 1088, the relative positions of the guide handle 1044 and the bending handle 1064 are fixed and maintained. The present disclosure does not limit the structure of the auxiliary support 108.

Referring to FIGS. 1 to 5, the interventional injection device 100A includes an operating handle 20, a sheath 30 and an injection member 40. The operating handle 20 includes a housing 22 and a driving assembly 24. The driving assembly 24 includes a driving knob 242 that is rotatably connected to the housing 22 and a driving rod 244 that is movably inserted into the housing 22 and is threaded to the driving knob 242. The proximal end of the sheath 30 is fixedly connected to the housing 22. The sheath 30 is movably inserted into the bending handle 1064 and the bendable sheath 1062 of the bending device 106. The injection member 40 is movably inserted into the sheath 30.

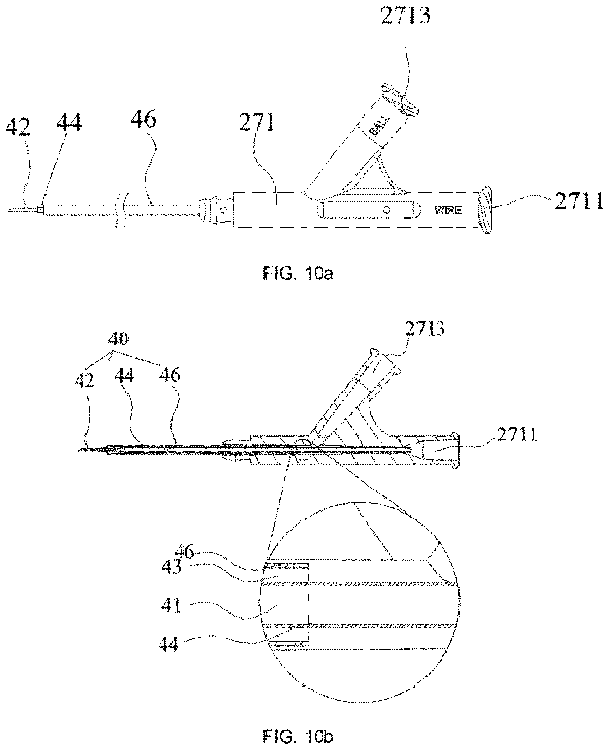

The injection member 40 includes a first channel 41 (as shown in FIG. 10b) and an injection needle 42 connected to the distal end of the first channel 41 in a tight seal. The proximal end of the injection member 40 is fixedly connected to the driving rod 244. The driving rod 244 is configured to be driven by the rotation of the driving knob 242 relative to the housing 22 to move axially, so as to drive the injection member 40 to move, so that the injection needle 42 can penetrate into the target tissue region.

When the injection needle 42 is required to move from the proximal end to the distal end (to advance the needle), for example, when the injection needle 42 is required to penetrate into the target tissue region, the driving knob 242 is operated to rotate in the first rotation direction (for example, clockwise), and thus the driving rod 244 is driven to move axially in a straight line from the proximal end to the distal end.

When the injection needle 42 is required to move from the distal end to the proximal end (to withdraw the needle), for example, when the injection needle 42 is required to move out from the target tissue region, the driving knob 242 is operated to rotate in a second rotation direction opposite to the first rotation direction (for example, counterclockwise), and thus the driving rod 244 is driven to move axially in a straight line from the distal end to the proximal end.

Traditional interventional injection devices usually control the injection needle to penetrate based on ejection or push-pull techniques. The ejection technique has extremely high requirements on the coaxiality of a plurality of tubes that form a nested tubular assembly. When the coaxiality requirements are not met, the injection needle, when being ejected, is prone to collide with the sheath, damaging and blunting the tip of the injection needle in severe cases, thereby affecting the accuracy of the surgery. The push-pull technique has poor controllability, and it is difficult to control the forward and backward speed of the needle through the handle when advancing and withdrawing the needle.

In the interventional injection device 100A according to the present disclosure, the driving rod 244 and the driving knob 242 are threaded to each other, to convert a rotational motion into a linear motion for advancing or withdrawing the injection needle 42. During operation, the advance and withdrawal speed of the needle can be controlled more accurately, effectively and stably. Further, the threaded connection has a certain self-locking property, so that backout of the injection needle 42 can be effectively prevented when the driving rod 244 drives the distal injection needle 42 to penetrate into the target tissue region, and the stability of the needle advancing process is further improved, improving the accuracy and efficiency of surgery.

In this embodiment, the housing 22 includes two mutually snap-fastenable first housings 2200, 2210. The first housings 2200, 2210 have a clamping position 2220 where the corresponding clamping member 1088 can clamp and engage with the first housings 2200, 2210.

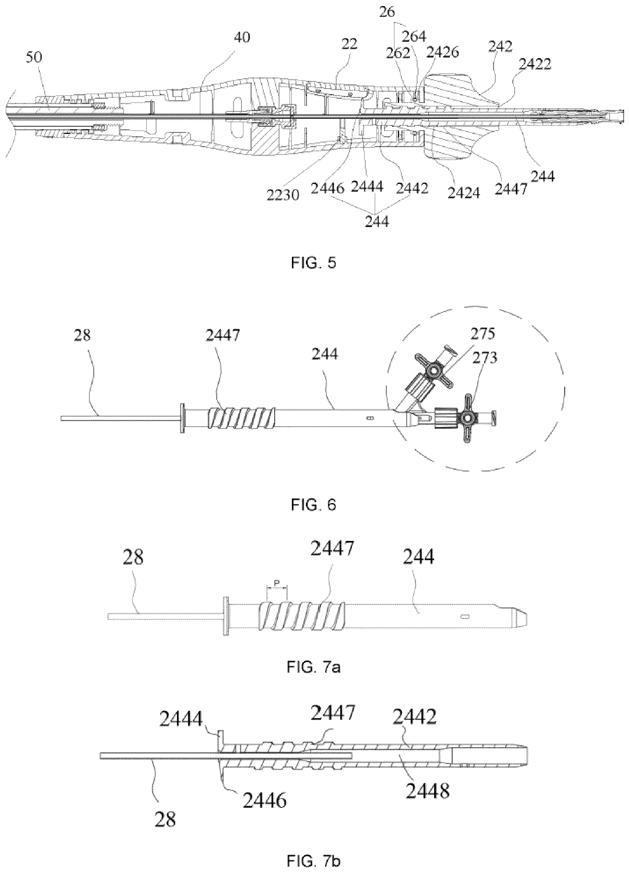

The driving knob 242 is rotatably connected to the proximal end of the housing 22 and is partially exposed outside the housing 22. The driving knob 242 can only rotate relative to the housing 22 but cannot move axially. The driving knob 242 defines an axially extending threaded hole 2422 for the driving rod 244 to pass through. The wall of the threaded hole 2422 is provided with a female thread 2424 (as shown in FIG. 5) for threaded connection with the driving rod 244.

Referring to FIG. 5, FIG. 6, FIG. 7a and FIG. 7b, the driving rod 244 includes a rod body 2442, a limiting portion 2444 and an indicator portion 2446. The outer wall of the rod body 2442 is provided with a male thread 2447. The rod body 2442 is inserted into the threaded hole 2422. The female thread 2424 of the driving knob 242 is threaded to the male thread 2447 of the rod body 2442. The male thread 2447 of the rod body 2442 can be a rectangular thread, a trapezoidal thread, a sawtooth thread, or the like. In this embodiment, the male thread 2447 of the rod body 2442 is preferably a trapezoidal thread. The trapezoidal thread has high root strength. The female thread 2424 of the threaded hole 2422 and the male thread 2447 of the rod body 2442 fit with each other through conical surfaces, with good centering performance, and are not susceptible to loosening, meeting usage needs better.

In this embodiment, when the driving knob 242 is rotated in the first rotation direction, the injection member 40 connected to the driving rod 244 moves distally. In an application scenario, before operating the driving knob 242, the safe distance between the injection needle 42 and the most distal end of the sheath 30 is about 7.5 mm, and the penetration depth of the injection needle 42 into the target tissue region is about 4.5 mm. The pitch P of the trapezoidal thread is approximately 8 millimeters (mm), and the thread is a single-start thread. One complete rotation of the driving knob 242 enables the driving rod 244 to drive the injection needle 42 to move a distance of L = P * 1 = 8 mm. Then, one and a half rotations of the driving knob 242 in the first rotation direction can cause the injection needle 42 to penetrate into the target tissue region at a suitable depth.

In other embodiments of the present disclosure, the safe distance between the injection needle 42 and the most distal end of the sheath 30 is not limited, the range of the thread pitch is not limited, and the moving distance of the injection needle 42 driven by the driving rod 244 through one complete rotation of the driving knob 242 is not limited.

The limiting portion 2444 radially protrudes from the outer wall of the rod body 2442. A blocking part 2230 (as shown in FIG. 5) is provided on the inner wall of the housing 22. The blocking part 2230 functions to block the limiting portion 2444 to limit the axial moving distance of the driving rod 244. When the driving rod 244 moves distally until the limiting portion 2444 is blocked by the blocking part 2230, it means that the driving rod 244 has reached a preset limit position and cannot continue moving distally, thereby controlling the injection depth of the injection needle 42 within a reasonable range.

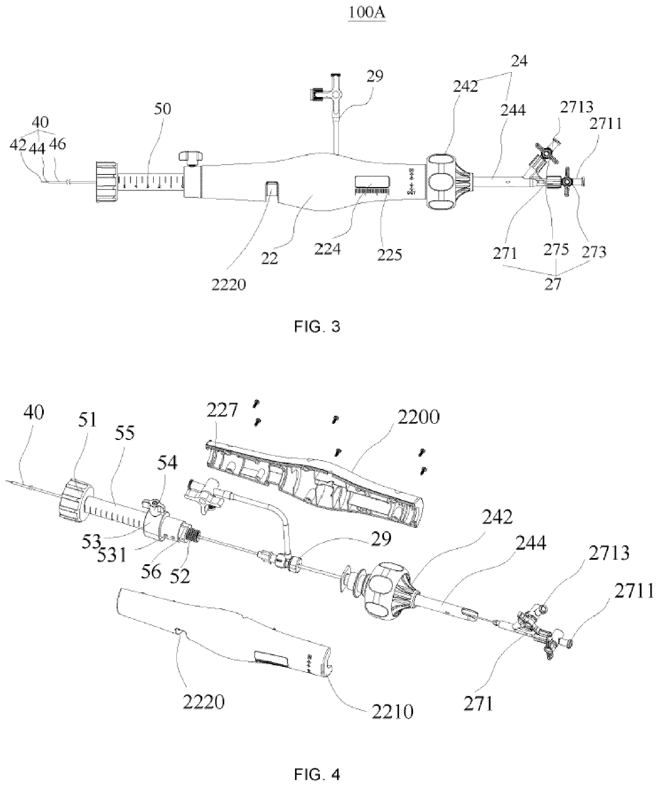

The indicator portion 2446 (shown in FIGS. 5 and 7b) radially protrudes from the outer wall of the rod body 2442, and the housing 22 is provided with a viewing window 224 (shown in FIG. 3) to show the indicator portion 2446. The housing 22 is further provided with a scale 225 (as shown in FIG. 3) for indicating the position of the indicator portion 2446. The viewing window 224 can be a transparent area or a hollow area. When the driving rod 244 moves relative to the housing 22, the position of the indicator portion 2446 can be determined through the viewing window 224 and the scale 225, so that the operator can determine the movement distance of the distal injection needle 42. In this way, the injection needle 42 can be prevented from penetrating too deeply into the target tissue region, otherwise the surgery may fail. For example, in the case where the target tissue region is the ventricular wall, if the injection needle 42 penetrates into the myocardial tissue too deeply, it may puncture the ventricular wall.

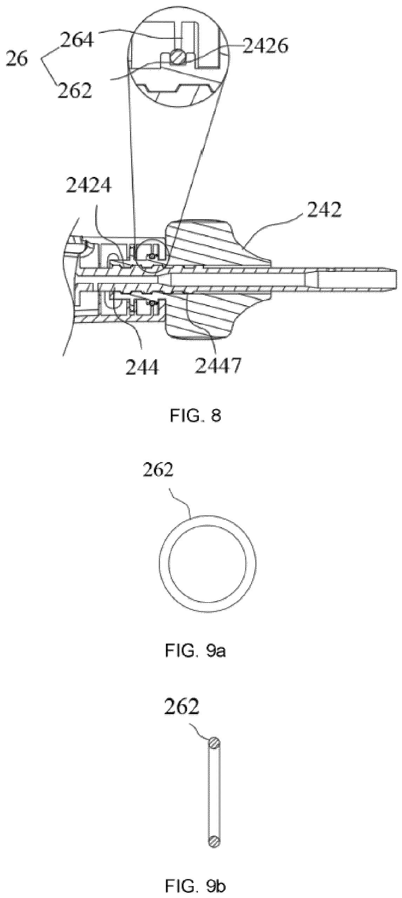

Referring to FIGS. 5 and 8, the operating handle 20 further includes a damping structure 26 located between the housing 22 and the driving knob 242, which functions to cooperate with the housing 22 to produce a damping effect during the rotation of the driving knob 242, to improve the handling feel of the driving knob 242 and make the rotation speed of the driving knob 242 more precise, thereby improving the uniformity and accuracy of the advance or withdrawal speed of the injection needle 42. The damping structure 26 includes a damping ring 262 and a rib 264 in an interference fit with the damping ring 262. In this embodiment, the rib 264 has a complete annular structure that is provided on the housing 22 and protrudes toward the interior of the housing. The driving knob 242 defines a circumferential groove 2426, and the circumferential groove 2426 is located in the housing 22. In other words, part of the driving knob 242 extends into the interior of the housing 22 and defines the circumferential groove 2426. The damping ring 262 is at least partially received in the circumferential groove 2426, facilitating the assembly of the damping ring 262 on the driving knob 242.

In this embodiment, the damping ring 262 is preferably but not limited to silicone, having good tensile strengths and tear resistances. The present disclosure does not limit the material of the damping ring 262, and for example, the material of the damping ring 262 can be rubber, provided that the damping ring 262 can provide a damping effect.

The damping effect of the damping structure 26 is related to the cross-sectional shape of the damping ring 262 and the interference amount between the damping ring 262 and the rib 264. The radial cross-sectional shape of the damping ring 262 can be rectangular, circular, trapezoidal or other special shapes. In this embodiment, the damping ring 262 is an O-ring (see FIG. 9a), and the radial cross section of the damping ring 262 is circular (see FIG. 9b), facilitating the manufacture. In addition, the damping ring 262 with a circular radial cross section can be assembled conveniently, without considering the installation direction. Depending on the magnitude of the damping, the interference amount on one side can be set in the range of 0.1 mm to 0.5 mm. In this embodiment, the interference amount on one side is preferably 0.25 mm, to ensure that the operator has more comfortable and good damping feel when rotating the driving knob 242, and thus to ensure an appropriate withdrawal speed of the needle. In other embodiments of the present disclosure, the range of the interference amount is not limited.

In other embodiments of the present disclosure, one of the damping ring 262 and the rib 264 is provided on the housing 22, and the other is provided on the driving knob 242. In other embodiments of the present disclosure, the rib can be consisted of a plurality of arc-shaped sections which are spaced from each other.

Referring to FIGS. 3, 4, 6, 10a and 10b, the operating handle 20 further includes an injection interface seat 27 located outside the housing 22. In this embodiment, the injection interface seat 27 is generally Y-shaped. The injection interface seat 27 is fixedly connected to the proximal end of the driving rod 244 and the proximal end of the injection member 40 is fixedly connected to the injection interface seat 27. The injection interface seat 27 includes a seat body 271, a first valve 273 and a second valve 275. In other embodiments of the present disclosure, the shape of the injection interface seat 27 is not limited.

The seat body 271 is fixedly connected to the proximal end of the rod body 2442. The seat body 271 and the rod body 2442 can be formed in one piece or separate pieces. In this embodiment, the seat body 271 and the driving rod 244 are formed in separate pieces, so that the structural complexity of the components can be reduced, facilitating the processing and manufacturing of the components, and the injection interface seat 27 and the driving rod 244 can be separately modularized and be conveniently assembled together. The injection interface seat 27 and the rod body 2442 can be connected by snapping, ultrasonic welding, glue bonding, etc. In this embodiment, the seat body 271 and the rod body 2442 are bonded with medical glue, providing better sealing effect.

The injection member 40 further includes a second channel 43 that is isolated from the first channel 41. The seat body 271 includes a first branch interface 2711 and a second branch interface 2713 that are isolated from each other. The first branch interface 2711 and the second branch interface 2713 are both connected to the injection member 40. The first branch interface 2711 and the second branch interface 2713 can be connected to different syringes. The first branch interface 2711 is selectively communicated to the first channel 41 and is used to provide a first injectable substance to the injection member 40. The second branch interface 2713 is selectively communicate to the second channel 43 and is used to provide a second injectable substance to the injection member 40. In this embodiment, the first injectable substance can be a myocardial repair substance, and the second injectable substance can be a contrast medium or other drugs. In other embodiments of the present disclosure, the functions and ingredients of the first injectable substance and the second injectable substance are not limited.

The first valve 273 is provided on the seat body 271 and is used to control the opening or closing of the first branch interface 2711. The second valve 275 is provided on the seat body 271 and is used to control the opening or closing of the second branch interface 2713. By operating the first valve 273, the first branch interface 2711 can be communicated to its corresponding syringe in one-way or be closed. By operating the second valve 275, the second branch interface 2713 can be communicated to its corresponding syringe in one-way or be closed.

Referring to FIG. 6, FIG. 7a and FIG. 7b again, the rod body 2442 defines a connection through-hole 2448 extending in the axial direction. The operating handle 20 further includes a first protection tube 28 inserted into the connection through-hole 2448 for the injection member 40 to pass through. The material of the first protection tube 28 includes but is not limited to stainless steel.

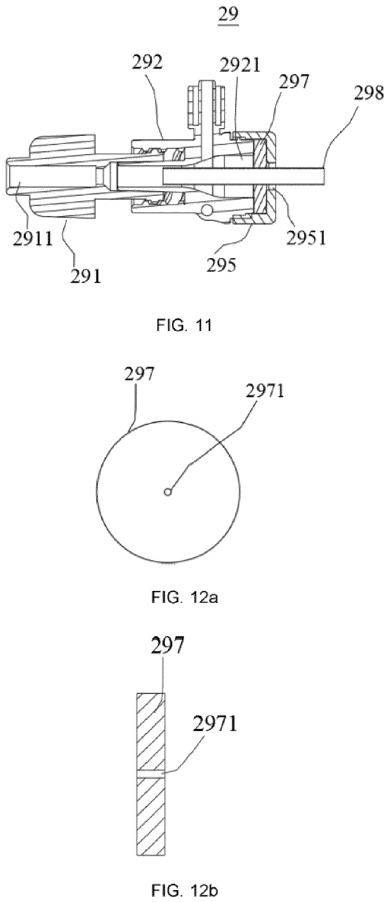

Referring to FIG. 4 and FIG. 11, the operating handle 20 further includes a hemostatic valve assembly 29 fixedly accommodated in the housing 22 for preventing blood leakage. The distal end of the hemostatic valve assembly 29 is fixedly connected to the proximal end of the sheath 30. In this embodiment, the hemostatic valve assembly 29 and the driving assembly 24 are coaxially installed in the housing 22 from distal end to proximal end. In other embodiments of the present disclosure, the hemostatic valve assembly 29 and the driving assembly 24 may not be coaxially installed in the housing 22.

The hemostatic valve assembly 29 includes a first joint 291, a second joint 292, a gasket cap 295, a sealing gasket 297 and a second protection tube 298.

The first joint 291 is fixedly accommodated in the housing 22. The first joint 291 defines a first installation hole 2911 extending in the axial direction for fixing the proximal end of the sheath 30. The first joint 291 can be, but is not limited to, a Luer fitting.

The second joint 292 is fixed to the housing 22 and partially accommodated in the housing 22. The second joint 292 is fixedly connected to the proximal end of the first joint 291. The second joint 292 defines a second installation hole 2921 extending in the axial direction for the second protection tube 298 to pass through. The second joint 292 can be, but is not limited to, a T-shaped joint.

The gasket cap 295 is fixed to the proximal end of the second joint 292. The sealing gasket 297 is received in the gasket cap 295. The gasket cap 295 defines a third installation hole 2951. The sealing gasket 297 is clamped between the proximal end surface of the second joint 292 and the inner wall of the gasket cap 295. Referring to FIG. 12a and FIG. 12b, the sealing gasket 297 defines a through hole 2971 for the second protection tube 298 to pass through, which is connected with the second protection tube 298 in a tight seal.

The second protection tube 298 passes through the third installation hole 2951, the through hole 2971, and the second installation hole 2921. In this embodiment, the second protection tube 298 and the sealing gasket 297 have an interference fit. The material of the second protection tube 298 includes but is not limited to stainless steel. Since the sealing gasket 297 needs to directly contact the blood, the material of the sealing gasket 297 is preferably silicone. The hardness of silicone is in the range of 20 A to 40 A. In this embodiment, the hardness of silicone is preferably 30 A. The sealing gasket 297 with this hardness has a certain compressive strength and fits closely with the second protection tube 298 to prevent blood leakage. The through hole 2971 of the sealing gasket can be a square hole, a rectangular hole, a polygonal hole, a conical hole or a round straight hole, etc. The cross section of the second protection tube 298 fits with the through hole 2971 to improve the closeness between the sealing gasket 297 and the second protection tube 298. For example, if the cross section of the second protection tube 298 is circular, then the shape of the through hole 2971 of the sealing gasket 297 is also circular.

As the injection member 40 would be introduced into the human body, the remaining part of the injection member 40 except the injection needle 42 should be flexible so that the injection member 40 can conform to the curved human body lumen. The hard first protection tube 28 can protect the proximal end of the injection member 40 and prevent the proximal end of the injection member 40 from being bent. The injection member 40 passes through the hard second protection tube 298 to prevent the injection member 40 from being pressed by the sealing gasket 297 and to prevent the injection member 40 from being bent due to too much resistance from the sealing gasket 297 when the injection member 40 passes through the sealing gasket 297, allowing the injectable substance to flow smoothly to the distal end during injection.

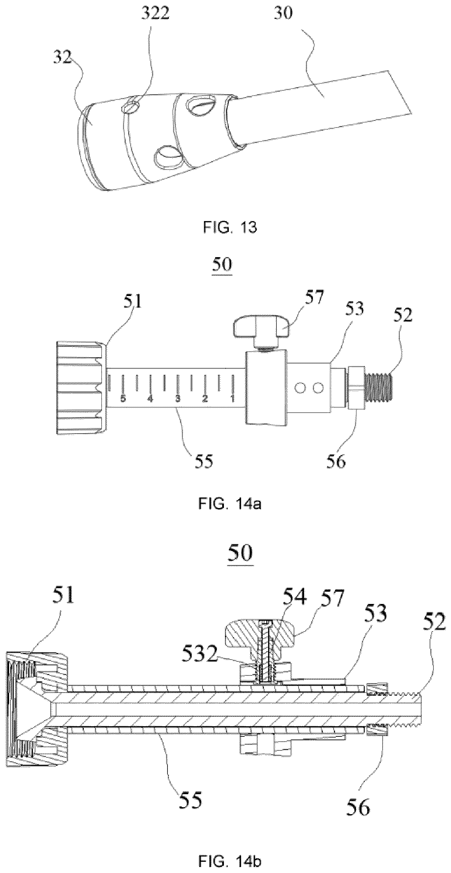

Referring to FIG. 3, FIG. 11 and FIG. 13, the proximal end of the sheath 30 is fixed to the first installation hole 2911 of the first joint 291 and inserted into the housing 22. The distal end of the sheath 30 is exposed out from the distal end of the housing 22. Referring to FIG. 3 and FIG. 13, the distal end of the sheath 30 is provided with a head 32 for contacting the target tissue region. The head 32 defines an injection orifice 322. The injection orifice 322 is communicated with the second channel 43 and is used for injecting the second injectable substance. The second injectable substance in the second channel 43 can reach the target tissue region (for example, in the cardiac cavity) through the injection orifice 322 for imaging or drug release. Preferably, the axial direction of the injection orifice 322 is perpendicular to the axial direction of the sheath 30, and a plurality of injection orifices 322 are defined at intervals in the circumferential direction of the head 32.

The operating handle 20 functions to adjust the position of the flexible injection member 40 and the position of the head 32 against the target tissue region (the inner wall of the ventricle), provide connection interfaces for the injectable substances, and advance the needle for penetrating.

After the bendable sheath 1062 is adjusted to an appropriate position, the operating handle 20 can be pushed distally to make the head 32 closely contact the target tissue region (for example, the endocardium), and the injection needle 42 can be advanced by the driving assembly 24 of the operating handle 20 to penetrate into the target tissue region to perform injection.

Referring to FIG. 3, FIG. 10a and FIG. 10b, the injection member 40 further includes an inner tube 44 and an outer tube 46 surrounding the inner tube 44. The distal end of the inner tube 44 is connected to the injection needle 42 in a tight seal. The lumen of the inner tube 44 forms the first channel 41. The inner tube 44 is fixedly inserted into the seat body 271 and communicates with the first branch interface 2711.

The outer tube 46 is fixedly inserted into the seat body 271 and communicates with the second branch interface 2713. The outer tube 46 surrounds the inner tube 44, and the second channel 43 is defined between the inner wall of the outer tube 46 and the outer wall of the inner tube 44. The injection needle 42 is exposed outside the distal end of the outer tube 46, without affecting the penetrating of the injection needle 42.

In order to adapt to the curved interventional path, the inner tube 44 and the outer tube 46 are preferably flexible tubes. The inner tube 44 and the outer tube 46 both pass through the first protection tube 28 and the second protection tube 298. The first protection tube 28 and the second protection tube 298 can protect the flexible inner and outer tubes 44 and 46, reducing the risk of the inner tube 44 and the outer tube 46 being damaged by bending.

Since the seat body 271 and the driving rod 244 are fixedly connected together, after the inner tube 44 and the outer tube 46 are fixedly connected to the seat body 271, the injection member 40 and the driving rod 244 are connected in one piece. Therefore, the axial movement of the driving rod 244 can synchronously push the distal injection needle 42 to penetrate the target tissue region, for example, to penetrate the endocardium or the ventricular wall.

In other embodiments of the present disclosure, the injection member 40 can be a dual-lumen tube or a multi-lumen tube including the first channel 41 and the second channel 43.

The fluid input via the first branch interface 2711 is transported to the distal end of the injection needle 42 via the first channel 41 in the inner tube 44; and the fluid input via the second branch interface 2713 is transported to the distal end of the injection needle 42 through the second channel 43 between the outer tube 46 and the inner tube 44. That is, both the fluid input via the first branch interface 2711 and the fluid input via the second branch interface 2713 are output from the distal end of the injection needle 42, and there are two fluid channels in the injection member 40.

Further, the injection member 40 is movably inserted into the sheath 30, and a third channel 45 is formed between the inner wall of the sheath 30 and the outer wall of the outer tube 46. The proximal end of the third channel 45 communicates with the hemostatic valve assembly 29, and the distal end communicates with needle outlet of the head 32 (located on the distal end surface of the head 32) and the injection orifices 322 on the peripheral wall.

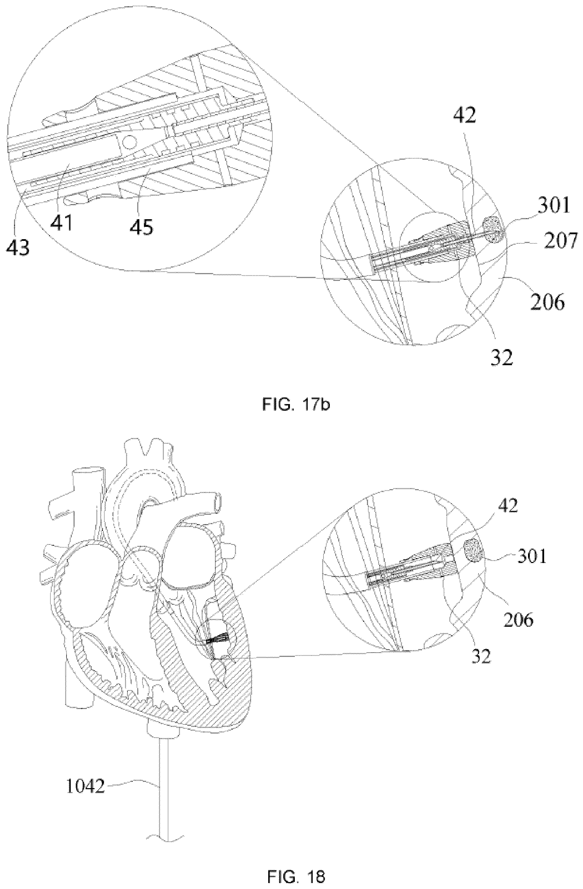

In summary, three fluid channels (the first channel 41, the second channel 43, and the third channel 45) are formed by the sheath 30 and the injection member 40 and different fluids can be transported therethrough as needed (see FIG. 17b).

Referring to FIGS. 3, 4, 14a and 14b, the interventional injection device 100A further includes an adjusting device 50 connected between the housing 22 and the bending handle 1064 for preventing or allowing the axial movement of the operating handle 20 relative to the bending handle 1064. The adjusting device 50 includes a connecting piece 51, a guide tube 52, a middle piece 53 and an adjusting piece 54.

The connecting piece 51 is fixedly connected to the proximal end of the bending handle 1064 and is used to connect the adjusting device 50 and the bending handle 1064 together.

The guide tube 52 is inserted into the connecting piece 51. The sheath 30 is movably inserted into the guide tube 52. The guide tube 52 is used to guide the sheath 30 into the bending handle 1064.

The middle piece 53 is movably arranged around the guide tube 52 and is fixedly connected to the housing 22. In this embodiment, an installation hole 531 is defined on the proximal end of the middle piece 53. A protruding installation post 227 is provided on the side wall of the inner cavity of the housing 22. The installation post 227 is engaged in the installation hole 531, thereby fixing the middle piece 53 to the housing 22. In other embodiments of the present disclosure, the fixed connection between the middle piece 53 and the housing 22 is not limited. For example, the middle piece 53 and the housing 22 can be fixed by gluing.

The adjusting piece 54 is movably provided on the middle piece 53 and is used to directly or indirectly lock or unlock the guide tube 52 in the radial direction of the middle piece 53 to prevent or allow the axial movement of the middle piece 53 relative to the guide tube 52, thereby preventing or allowing the axial movement of the operating handle 20 relative to the bending handle 1064, in order to adjust the length of the distal end of the sheath 30 extending beyond the distal end of the bendable sheath 1062.

In this embodiment, when the bottom of the adjusting piece 54 passes through the middle piece 53 and abuts against the guide tube 52, the guide tube 52 cannot move relative to the middle piece 53, and thus the operating handle 20 cannot move relative to the bending handle 1064, thereby fixing the position of the distal head 32 of the sheath 30 relative to the distal end of the bendable sheath 1062.

In this embodiment, when the adjusting piece 54 unlocks the guide tube 52, there is a gap between the adjusting piece 54 and the guide tube 52, the guide tube 52 can move relative to the middle piece 53, and thus the operating handle 20 can move axially relative to the bending handle 1064, so that the length of the distal head 32 of the sheath 30 extending beyond the distal end of the bending handle 1064 can be adjusted. When the adjusting piece 54 unlocks the guide tube 52, if the operating handle 20 is rotated, the operating handle 20 can drive the sheath 30 and the injection member 40 to rotate synchronously.

In this embodiment, the middle piece 53 defines an adjustment hole 532 extending in the radial direction. The adjustment hole 532 is a threaded hole. The adjusting piece 54 is inserted into the adjustment hole 532 and threaded with the adjustment hole 532 so that it can move in the adjustment hole 532.

The adjusting device 50 further includes a scale tube 55 and a positioning piece 56. The scale tube 55 is arranged around the guide tube 52. The scale tube 55 is located between the middle piece 53 and the guide tube 52. The positioning piece 56 is connected to the proximal end of the guide tube 52 so that the scale tube 55 is limited between the connecting piece 51 and the positioning piece 56 in the axial direction. In this embodiment, the proximal end of the guide tube 52 is threaded with the positioning piece 56. The scale tube 55 has scales in the axial direction to show the movement distance of the operating handle 20.

It can be understood that since the scale tube 55 is added outside the guide tube 52, the adjusting piece 54 indirectly abuts against or releases the guide tube 52 when the bottom of the adjusting piece 54 passes through the middle piece 53 to abut against or releases the scale tube 55.

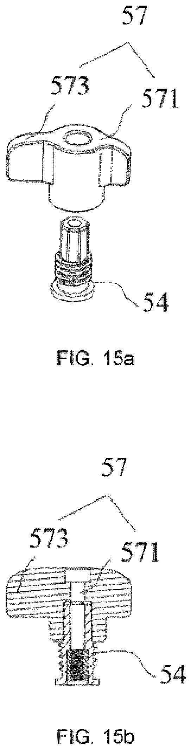

Referring to FIG. 15a and FIG. 15b, the adjusting device 50 further includes an adjustment knob 57 connected to the adjusting piece 54 wherein the adjustment knob 57 and the adjusting piece 54 cannot rotate relative to each other, and is used to drive the adjusting piece 54 to move in the radial direction of the middle piece 53. The adjustment knob 57 includes a base 571 and an adjustment indicator 573 provided on the outer wall of the base 571. The base 571 is arranged around the adjusting piece 54 and they cannot rotate relative to each other. In this embodiment, at least one pair of mating planes is provided between the base 571 and the adjusting piece 54 to prevent relative rotation therebetween. For example, the base 571 is provided with an octagonal hole, the top of the adjusting piece 54 has an octagonal cross-section, and the top of the adjusting piece 54 is engaged with the octagonal hole so that the adjusting piece 54 and the base 571 cannot rotate relative to each other.

The adjustment indicator 573 protrudes from the outer peripheral wall of the base 571 in the radial direction of the base 571, and the extension length thereof is different from that of the other part of the adjustment knob 57. In this embodiment, the adjustment indicator 573 has a longer extension length. When the adjustment indicator 573 points to a predetermined direction or when the adjustment indicator 573 rotates a predetermined angle, for example, 90°, it can be determined that the adjusting piece 54 has abutted against the scale tube 55.

When the adjustment knob 57 is loosened, there is a gap between the bottom of the adjusting piece 54 and the scale tube 55. Now, the operating handle 20 and the scale tube 55 can be operated to move along the scale tube 55 to be relatively close to or away from the bending handle 1064, thereby adjusting the distance of the distal head 32 of the sheath 30 relative to the distal end of the bendable sheath 1062. When the adjustment knob 57 is tightened, the bottom of the adjusting piece 54 abuts against the scale tube 55, and the operating handle 20 cannot move relative to the bending handle 1064, thereby fixing the position of the distal head 32 of the sheath 30 relative to the distal end of the bendable sheath 1062.

Taking the left ventricular wall as the target tissue region for example, the interventional injection system 1000A can inject the myocardial repair substance into the myocardial tissue of the left ventricular wall via a suitable interventional path, preferably via the femoral artery-aortic arch-aorta-left ventricular path. Taking this interventional path as an example, in surgery, the femoral artery is first punctured, the guiding sheath 1042 first establishes a passage to the left ventricle, then the bendable sheath 1062 is introduced into the left ventricle along the interior lumen of the guiding sheath 1042, and thereafter the bendable sheath 1062 is adjusted to a suitable injection location, thereby complete the establishment of the injection path. Then the operating handle 20 is operated as follows.

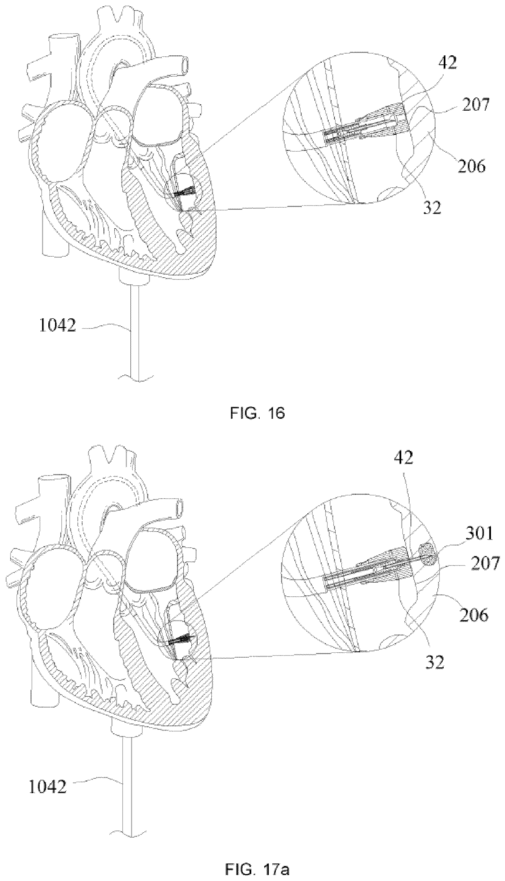

In step 1, the adjustment knob 57 is loosened, the entire operating handle 20 is slowly pushed forward to push the sheath 30 and the flexible injection member 40 forward, and under the guidance of DSA and ultrasound images, by determining the position of the head 32 of the sheath 30, it is determined whether the sheath 30 has contacted the surface of the left ventricular wall 206, for example, the endocardium 207 (which can be regarded as the surface of the target tissue region) in the left ventricle, as shown in FIG. 16.

In step 2, after determining that the head 32 has contacted the endocardium 207, the driving knob 242 is rotated in the first rotation direction (for example, clockwise) to make the injection needle 42 penetrate the endocardium 207 into the left ventricular wall 206. When the driving rod 244 reaches the preset limit position as mentioned above, the driving knob 242 cannot continue rotating, and thus the injection needle 42 cannot advance further, thereby limiting the injection depth within a safe range, as shown in FIGS. 17a and 17b.

In step 3, after determining that the injection needle 42 has penetrated into the target injection site, the syringe can be used to inject the required first injectable substance 301 via the injection interface seat 27 into the left ventricular wall 206 through the first branch interface 2711 and the first channel 41. After the injection is finished, the injection needle 42 is withdrawn until it is separated from the left ventricular wall 206, as shown in FIG. 18. Also, a second injectable substance such as a contrast medium or other drugs can be injected through the second branch interface 2713, the second channel 43 and the injection orifices 322 on the distal head 32 of the sheath 30 into the left ventricle.

The above steps are repeated at a plurality of desired injection locations, and then the whole device is withdrawn, thereby completing the surgery.

The target tissue region can also be other myocardial tissues of the heart, such as the interventricular septum, the ventricular wall of the right ventricle, etc. Depending on different repair sites, corresponding interventional paths can be selected. For example, when the target tissue region is the ventricular wall of the right ventricle, then the interventional path can be the femoral vein-inferior vena cava-right atrium-right ventricular path.

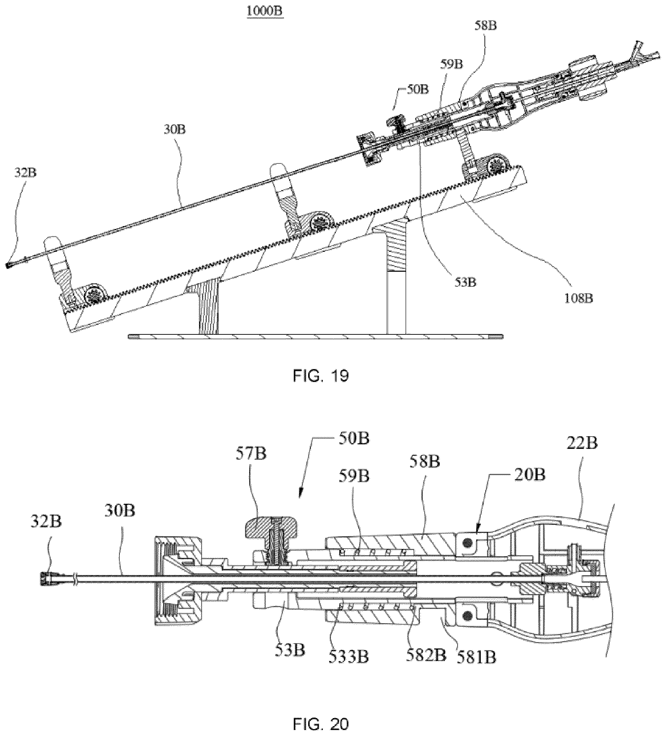

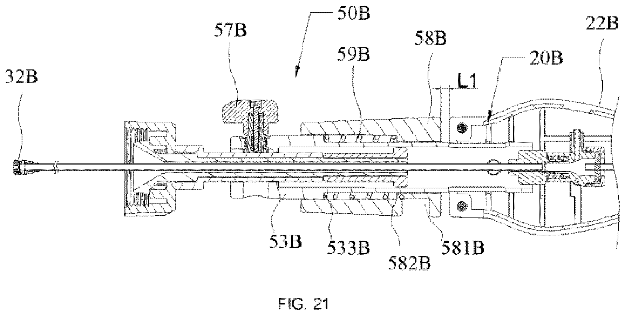

In other embodiments of the present disclosure, referring to FIG. 19, FIG. 20 and FIG. 21, in the interventional injection system 1000B, the adjusting device 50B further includes a sleeving tube 58B and a spring element 59B, to provide feedback to the operator when the head 32B of the sheath 30B contacts the target tissue region. That is, a feedback mechanism is provided, for prompting that the head 32B of the sheath 30B has contacted the target tissue region. This allows the operator to determine whether the head 32 has contacted the target tissue region without ultrasound images, simplifying the surgical process and improving the surgical efficiency.

The sleeving tube 58B is fixedly connected to the auxiliary support 108B. The sleeving tube 58B has a clamping position 581B where the corresponding clamping member on the auxiliary support 108B can clamp and fix the sleeving tube 58B, thereby fixedly connecting the sleeving tube 58B on the auxiliary support 108B. In other embodiments of the present disclosure, the sleeving tube 58B and the auxiliary support 108B are not necessarily connected by clamping. The sleeving tube 58B is movably arranged around the middle piece 53B. The outer wall of the middle piece 53B is provided with a first abutment portion 533B, and the inner wall of the sleeving tube 58B is provided with a second abutment portion 582B. The first abutment portion 533B includes, but is not limited to, a radial step on the middle piece 53B, and the second abutment portion 582B includes, but is not limited to, a radial step on the inner wall of the sleeving tube 58B.

The spring element 59B is arranged around the middle piece 53B and received in the sleeving tube 58B. In the initial state, the proximal end of the sleeving tube 58B abuts the distal end of the housing 22B. The spring element 59B is disposed between the middle piece 53B and the sleeving tube 58B. One end of the spring element 59B abuts the first abutment portion 533B, and the other end of the spring element 59B abuts the second abutment portion 582B.

When the distal head 32B of the sheath 30B contacts the target tissue region, if further force is applied to push the sleeving tube 58B distally, the spring element 59B will be pressed and deformed by the sleeving tube 58B and the middle piece 53B, and the head 32B, the middle piece 53B and the housing 22B are relatively fixed, while only the sleeving tube 58B moves distally. That is, the middle piece 53B moves proximally relative to the sleeving tube 58B, and a gap is formed between the proximal end of the sleeving tube 58B and the distal end of the housing 22B. Specifically, after adjusting the bendable sheath to an appropriate position, it is necessary to loosen the adjustment knob 57B and push the entire operating handle 20B to move forward (this pushing step is completed by directly applying force on the sleeving tube 58B through the auxiliary support 108B), so that the head 32B of the sheath 30B extends out of the distal end of the bendable sheath. When the sheath 30B moves until the head 32B contacts the target tissue region (for example, the ventricular wall), the head 32B cannot continue advancing forward, and the target tissue region exert a backward reaction force to the head 32B, which force is transmitted to the operating handle 20B and the middle piece 53B fixedly connected to the operating handle 20B through the sheath 30B. If the operator continues pushing the sleeving tube 58B forward, the reaction force from the head 32B will cause the first abutment portion 533B and the second abutment portion 582B to move toward each other, that is, the spring element 59B will be compressed by the force, and the middle piece 53B and the operating handle 20B will move proximally relative to the sleeving tube 58B fixed by the clamping member, thereby separating the sleeving tube 58B from the distal end of the housing 22B of the operating handle 20B with a gap L1 formed therebetween, prompting that the head 32B has reached the target tissue region and a further needle advance operation can be performed.

In the interventional injection system of the present disclosure, the distal head has a multi-channel structure, combined with the feedback mechanism on the operating handle, the process and effect of the head contacting tissue can be determined timely and accurately, and the fluid type can be switched timely as needed. In a specific case of multi-point injection, the orientation and position of the injection needle can be changed quickly by the rotation of the entire guide handle and the entire bending handle as well as the bendable characteristic, avoiding or alleviating repeated axial adjustment of the tubes, greatly shortening the injection time and improving the surgical efficiency.

The above embodiments are only preferred embodiments of the present disclosure and do not limit the present disclosure in any form. While the disclosure has been particularly shown and described in conjunction with the above preferred embodiments, these embodiments are not intended to limit the present disclosure. Those skilled in the art can make various modifications and variations to the technical solutions of the present disclosure motivated by the methods and techniques disclosed above, or obtain equivalent embodiments without departing from the scope of the disclosure. Thus, any simple amendments, equivalent changes and modifications made to the above embodiments under the teaching of the present disclosure without departing from the spirit of the present disclosure still fall within the scope of the present disclosure.

## Claims

1. An interventional injection device, comprising an operating handle, a sheath and an injection member;
wherein the operating handle comprises a housing and a driving assembly; the driving assembly comprises a driving knob that is rotatably connected to the housing and a driving rod that is movably inserted into the housing and threaded to the driving knob;
a proximal end of the sheath is fixedly connected to the housing;
the injection member is movably inserted in the sheath, the injection member comprises a first channel and an injection needle connected to a distal end of the first channel in a tight seal, and a proximal end of the injection member is fixedly connected to the driving rod; and
the driving rod is configured to move axially by rotation of the driving knob relative to the housing so as to drive the injection member to move and thus drive the injection needle to penetrate into a target tissue region.

2. The interventional injection device of claim 1, wherein the driving knob defines a threaded hole extending axially, an inner wall of the threaded hole is provided with a female thread, an outer wall of the driving rod is provided with a male thread, and the driving rod passes through the threaded hole so that the female thread is threaded with the male thread.

3. The interventional injection device of claim 2, wherein the driving rod comprises:
a rod body, the male thread is provided on the rod body; and
a limiting portion, which radially protrudes from an outer wall of the rod body;
wherein an inner wall of the housing is provided with a blocking part which is configured to block the limiting portion so as to limit axial movement of the driving rod.

4. The interventional injection device of claim 3, wherein the driving rod further comprises an indicator portion which radially protrudes from the outer wall of the rod body; and
the housing is provided with a viewing window to show the indicator portion, and the housing is provided with a scale for indicating position of the indicator portion.

5. The interventional injection device of claim 1, wherein a damping structure is provided between the driving knob and the housing.

6. The interventional injection device of claim 5, wherein the damping structure comprises a damping ring and a rib in an interference fit with the damping ring, and one of the damping ring and the rib is provided on the housing, and an other is provided on the driving knob.

7. The interventional injection device of claim 6, wherein a part of the driving knob extends into an interior of the housing and is provided with a circumferential groove, and the damping ring is at least partially received in the circumferential groove; and the rib protrudes inward on an inner wall of the housing, and the rib press the damping ring.

8. The interventional injection device of any one of claims 1 to 7, further comprises an injection interface seat located outside the housing, a proximal end of the driving rod is fixedly connected to the injection interface seat, and a proximal end of the injection member is fixedly connected to the injection interface seat.

9. The interventional injection device of claim 8, wherein the driving rod defines a connection through-hole extending in an axial direction, a first protection tube is arranged in the connection through-hole and extends out of the connection through-hole, and the injection member is inserted into the first protection tube and the connection through-hole.

10. The interventional injection device of any one of claims 1 to 7, wherein the operating handle further comprises a hemostatic valve assembly fixedly installed in the housing, and a distal end of the hemostatic valve assembly is fixedly connected to the proximal end of the sheath, wherein the hemostatic valve assembly comprises a sealing gasket, a second protection tube passes through the sealing gasket, the second protection tube is connected to the sealing gasket in a tight seal, and the injection member passes through the second protection tube.

11. The interventional injection device of claim 8, wherein the injection member further comprises a second channel that is isolated from the first channel.

12. The interventional injection device of claim 11, wherein the injection interface seat comprises:
a first branch interface selectively connected to a proximal end of the first channel; and
a second branch interface selectively connected to a proximal end of the second channel;
wherein the second branch interface and the first branch interface are isolated from each other.

13. The interventional injection device of claim 11, wherein the injection member further comprises an inner tube and an outer tube surrounding the inner tube; a lumen of the inner tube forms the first channel, and the second channel is defined between an inner wall of the outer tube and an outer wall of the inner tube; and at least the injection needle is exposed outside a distal end of the outer tube.

14. The interventional injection device of claim 13, wherein a distal end of the sheath is provided with a head for abutting the target tissue region, the head defines an injection orifice, and a distal end of the second channel is communicated with the injection orifice.

15. The interventional injection device of claim 14, wherein the injection orifice has an axial direction perpendicular to an axial direction of the sheath, and a plurality of injection orifices are distributed on the head at intervals in a circumferential direction.

16. The interventional injection device of claim 14, wherein a third channel is defined between an inner wall of the sheath and an outer wall of the outer tube, a distal end surface of the head has a needle outlet, and a distal end of the third channel is communicated with the needle outlet and the injection orifice.

17. The interventional injection device of claim 16, wherein the operating handle further comprises a hemostatic valve assembly fixedly provided in the housing, and a proximal end of the third channel is communicated with the hemostatic valve assembly.

18. The interventional injection device of claim 14, further comprising a feedback mechanism, the feedback mechanism comprising:
a middle piece fixedly connected to the housing of the operating handle, an outer wall of the middle piece being provided with a first abutment portion;
a sleeving tube movably arranged around the middle piece, an inner wall of the sleeving tube is provided with a second abutment portion, wherein in an initial state, a proximal end of the sleeving tube contacts a distal end of the housing, and the second abutment portion is located on a proximal side of the first abutment portion; and
a spring element, one end of the spring element abuts the first abutment portion, and an other end of the spring element abuts the second abutment portion;
wherein when the distal end of the sheath abuts the target tissue region, the middle piece is forced to press the spring element and the middle piece moves proximally relative to the sleeving tube, generating a gap between the proximal end of the sleeving tube and the distal end of the housing.

19. The interventional injection device of claim 18, wherein the spring element is arranged around the middle piece and received in the sleeving tube.

20. The interventional injection device of claim 18, wherein the first abutment portion is a radial step radially provided on the middle piece.

21. The interventional injection device of claim 18, wherein the second abutment portion is a radial step radially provided on the inner wall of the sleeving tube.

22. An interventional injection system, comprising:
a bending device, comprising a bendable sheath and a bending handle for controlling the bendable sheath; and
the interventional injection device of any one of claims 1 to 17;
wherein the sheath is movably inserted in the bending device.

23. The interventional injection system of claim 22, further comprising an adjusting device, the adjusting device comprises:
a connecting piece, fixedly connected to a proximal end of the bending handle;
a guide tube, inserted into the connecting piece and rotatably connected to the connecting piece, the sheath being movably inserted into the guide tube;
a middle piece, movably arranged around the guide tube and fixedly connected to the housing; and
an adjusting piece, movably provided on the middle piece and configured to directly or indirectly lock or unlock the guide tube in a radial direction of the middle piece to prevent or allow axial movement of the middle piece relative to the guide tube, thereby preventing or allowing axial movement of the operating handle relative to the bending handle.

24. The interventional injection system of claim 23, further comprising an auxiliary support;
an outer wall of the middle piece being provided with a first abutment portion;
the adjusting device further comprising:
a sleeving tube fixedly connected to the auxiliary support and movably arranged around the middle piece, an inner wall of the sleeving tube being provided with a second abutment portion, wherein in an initial state, a proximal end of the sleeving tube contacts a distal end of the housing; and
a spring element provided between the middle piece and the sleeving tube, wherein one end of the spring element abuts the first abutment portion, and an other end of the spring element abuts the second abutment portion;
wherein when a distal end of the sheath abuts the target tissue region, the middle piece is forced to press the spring element and the middle piece moves proximally relative to the sleeving tube, generating a gap between the proximal end of the sleeving tube and the distal end of the housing.
